(19) Europäisches Patentamt / European Patent Office / Office européen des brevets

(11) **EP 3 116 383 B1**

(12) **EUROPEAN PATENT SPECIFICATION**

(45) Date of publication and mention of the grant of the patent:
**05.01.2022 Bulletin 2022/01**

(21) Application number: **14885237.9**

(22) Date of filing: **17.12.2014**

(51) Int Cl.:
*A61B 5/02* *(2006.01)*          *A61B 5/0295* *(2006.01)*
*A61B 5/022* *(2006.01)*          *A61B 5/00* *(2006.01)*

(86) International application number:
**PCT/US2014/070803**

(87) International publication number:
**WO 2015/138026 (17.09.2015 Gazette 2015/37)**

(54) **METHOD AND DEVICE FOR DETECTING AND ASSESSING REACTIVE HYPEREMIA USING SEGMENTAL PLETHYSMOGRAPHY**

VERFAHREN UND VORRICHTUNG ZUR ERKENNUNG UND BEWERTUNG REAKTIVER HYPERÄMIE ÜBER SEGMENTPLETHYSMOGRAPHIE

PROCÉDÉ ET DISPOSITIF PERMETTANT DE DÉTECTER ET D'ÉVALUER UNE HYPERÉMIE RÉACTIONNELLE, GRÂCE À LA PLÉTHYSMOGRAPHIE SEGMENTAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.03.2014 US 201414204736**

(43) Date of publication of application:
**18.01.2017 Bulletin 2017/03**

(73) Proprietor: **Cordex Systems, Inc.**
**Annapolis, MD 21401 (US)**

(72) Inventors:
• **WHITT, Michael David**
 **South Bend, IN 46614 (US)**
• **MAGLIATO, Kathy Elizabeth**
 **Pacific Palisades, CA 90272 (US)**
• **RITTERBUSH, Stephen**
 **Great Falls, VA 22066 (US)**

(74) Representative: **Forrest, Stuart et al**
**WP Thompson**
**138 Fetter Lane**
**London EC4A 1BT (GB)**

(56) References cited:
**US-A1- 2003 065 270      US-A1- 2005 070 805**
**US-A1- 2009 259 131      US-A1- 2010 305 459**
**US-A1- 2010 305 459      US-B1- 6 309 359**
**US-B1- 6 309 359**

• **Damiano Baldassarre ET AL: "Time course of forearm arterial compliance changes during reactive hyperemia", American Journal of Physiology - Heart and Circulatory Physiology, 1 September 2001 (2001-09-01), pages 1093-1103, XP055268086, United States Retrieved from the Internet: URL:http://ajpheart.physiology.org/content /281/3/H1093.full-text.pdf [retrieved on 2016-04-22]**

Description

RELATED APPLICATIONS

This application claims priority to U.S. Patent Application Serial No. 14/204,736, filed on March 11, 2014.

FIELD

The technology relates generally to medical devices and diagnosis methods and, in particular, to methods and devices for measuring reactive hyperemia and endothelial dysfunction with segmental volume plethysmography and oscillometry.

BACKGROUND

Endothelial dysfunction (ED) has been shown to be of prognostic significance in predicting vascular events such as heart attack and stroke. It is the key event in the development of atherosclerosis and predates clinically evident vascular pathology by many years. ED can result from a variety of disease processes, such as hypertension, atherosclerosis, cardiovascular disease (heart disease and stroke), atrial fibrillation, congestive heart failure, peripheral vascular disease, septic shock, hypercholesterolemia, type I and II diabetes, erectile dysfunction, rheumatic arthritis, HIV, liver disease (cirrhosis, hepatitis B and C, non-alcoholic steatohepatitis, fatty liver disease), pre-eclampsia, heat stress, all forms of dementia and psychological illness, any and all illness related to localized or systemic inflammation, environmental factors such as smoking, ingestion of high glycemic index carbohydrates, sedentary lifestyle and obesity. ED is also associated with states of low grade, chronic inflammation with elevated C reactive protein which leads to atherosclerosis.

ED can be improved by risk factor modification: exercise, weight loss, cessation of smoking, the use of statin drugs, beta blockade, the treatment of hypertension and hypercholesterolemia, improved diet with reduction of trans fat intake, control of diabetes. Therefore, early detection of ED may allow not only early diagnosis and treatment of ED-related diseases, but also treatment of ED itself.

Plethysmography is a non-invasive technique for measuring the amount of blood flow present or passing through, an organ or other part of the body. Segmental volume plethysmography is performed by injecting a standard volume of air into a pneumatic cuff or cuffs placed at various levels along an extremity. Volume changes in the limb segment below the cuff are translated into pulsatile pressure that are detected by a transducer and then displayed as a pressure pulse contour. Segmental volume plethysmography has been commonly used to measure blood volume change. It may also be used to check for blood clots in the arms and legs.

US2010/305459 A1 discloses measuring reactive hyperemia using a pressurized cuff to determine pressure-area (P-A) curves.

US 6309359 B1 discloses measuring arterial compliance using a cuff and a flow meter.

SUMMARY

One aspect of the present application relates to a method for measuring reactive hyperemia in a subject. The method comprises the steps of performing a first segmental cuff plethysmography having an inflation phase and a deflation phase; generating a baseline arterial compliance curve and/or a baseline pressure-area (P-A) curve on a portion of the body of the subject, wherein the cuff pressure is increased to a first peak cuff pressure during the inflation phase and immediately reduced from the first peak cuff pressure during the deflation phase; performing a second segmental cuff plethysmography having an inflation phase, a holding phase, and a deflation phase; generating a hyperemic arterial compliance curve and/or a hyperemic P-A curve, wherein the cuff pressure is increased to a second peak level during the inflation phase, maintained at the second peak cuff pressure for a predetermined period of time during the holding phase, and then reduced from the second peak cuff pressure during the deflation phase; calculating the difference between the baseline arterial compliance curve and the hyperemic arterial compliance curve as an area between the arterial compliance curves, and/or the difference between the baseline P-A curve and the hyperemic P-A curve as an area between the P-A curves; measuring a level of reactive hyperemia based on the area between the arterial compliance curves and/or the area between the P-A curves, wherein a first cuff compliance curve is generated during the first segmental cuff plethysmography based only on data collected during the deflation phase of the first segmental cuff plethysmography and a second cuff compliance curve is generated during the second segmental cuff plethysmography based only on data collected during the deflation phase.

Another aspect of the present application not covered by the present invention relates to a method for determining endothelial dysfunction (ED) in a subject. The method comprises the steps of (a) inflating a cuff around a portion of the body of the subject and immediately deflating the cuff after reaching a first cuff pressure and measuring the volume and pressure changes in the cuff during the deflation process; (b) generating a first curve based on the measurements in step (a); (c) inflating the cuff around the portion of the body of the subject for the second time, maintaining the inflation at a second cuff pressure for a predetermined period of time, deflating the cuff, and measuring the volume and pressure changes in the cuff during the deflation process; (d) generating a second curve based on the measurements in step (c); (e) determining a difference between areas under the first curve and the second curve, wherein the first curve and the second curve are arterial compliance curves or pressure-area (P-A) curves; and (f) determining a level of endothelial dysfunction based on the difference determined in step (e), wherein a first cuff compliance curve is generated during step (a) and a second cuff compliance curve is generated during step (c) using a flowmeter to directly measure the volume change in the cuff, and a pressure sensor to measure pressure change for each known volume change, wherein the first curve is generated based the first cuff compliance curve, and wherein the second curve is generated based the second cuff compliance.

Another aspect of the present application relates to an apparatus for measuring reactive hyperemia in a subject. The apparatus comprises: an inflatable cuff having an inlet and an outlet; a pump connected to said inlet of said cuff for inflating the cuff; a flowmeter connected to said outlet of said cuff; a pressure transducer for measuring the pressure inside the cuff; and a computer configured to: generate a cuff compliance curve by directly measuring volume change in said cuff with said pump and pressure change inside the cuff with said pressure transducer; calculate an arterial compliance curve, which includes a first arterial compliance curve and a second arterial compliance curve, and a pressure-area (P-A) curve, which includes a first P-A curve and a second P-A curve, only during the deflation process of the cuff; and calculate a difference between areas under the first arterial compliance curve and the second arterial compliance curve and a difference between areas under the first P-A curve and the second P-A curve.

## BRIEF DESCRIPTION OF THE DRAWINGS

The detailed description will refer to the following drawings, wherein like numerals refer to like elements, and wherein:

FIG. 1 is a block diagram illustrating an embodiment of a system for measuring arterial compliance, area and peripheral arterial flow.

FIG. 2 is a flow chart illustrating an embodiment of a method for measuring arterial compliance, area and peripheral arterial flow

FIG. 3 is a graph illustrating data acquired using an embodiment of the system and method for measuring arterial compliance, area and peripheral arterial flow.

FIG. 4 is a graph illustrating data acquired and filtered using an embodiment of the system and method for measuring arterial compliance, area and peripheral arterial flow

FIG. 5 includes graphs illustrating cuff compliance calculated using an embodiment of the system and method for measuring arterial compliance, area and peripheral arterial flow

FIG. 6 is a graph illustrating cuff compliance calculated using an embodiment of the system and method for measuring arterial compliance, area and peripheral arterial flow

FIG. 7 is a graph illustrating a metric applied using an embodiment of the system and method for measuring arterial compliance, area and peripheral arterial flow.

FIG. 8 is a graph illustrating a metric applied using an embodiment of the system and method for measuring arterial area.

FIG. 9 is a block diagram illustrating another embodiment of a system for measuring arterial compliance, area and peripheral arterial flow.

FIG. 10 is a schematic drawing of a printed circuit board (PCB) for the system of FIG. 9

FIG. 11 shows an exemplary cuff deflation curve. During the deflation phase, the cuff pressure typically decreased from about 200 mmHg to about 0 mmHg. The patient data is typically obtained at pressure above central venous pressure which is approximately 5 mmHg

FIG. 12 shows an exemplary band pass filter frequency response output.

FIG. 13 shows band pass filter (0.5 - 5.0 Hertz) of cuff descent data (top panel) and standard cuff descent data (bottom panel).

FIG. 14 shows exemplary pressure-time derivative and Doppler velocity waveforms.

FIG. 15 shows exemplary flow waveforms calculated from pressure-time derivatives.

## DETAILED DESCRIPTION

Described herein are methods, systems and devices for measuring arterial compliance, and generating other measurements such as arterial volumetric blood flow waveforms and pressure-area curves, over the entire transmural pressure range. These measurements can be used to measure reactive hyperemia and detect, measure and monitor various ailments such as endothelial dysfunction, other cardiovascular diseases, and pre-eclampsia as well as for monitoring the effectiveness or efficacy of anesthesia.

Embodiments include a mathematical function-calibrated cuff plethysmography. A plethysmograph is an instrument for determining and registering variations in the size of an organ or limb resulting from changes in the amount of blood present or passing through it. The calibration is achieved with a process that combines a non-linear mathematical function and the output of a metering pump. Embodiments combine concepts of segmental volume plethysmography and oscillometry to provide an actual measurement of arterial compliance over the entire transmural pressure range. Segmental volume plethysmography is a technique that is performed by injecting a standard volume of air into a pneumatic cuff or cuffs placed at various levels along an extremity. Volume changes in the limb segment underneath the cuff are translated into pulsatile pressure that are detected by a transducer and then displayed as a pressure pulse contour. Oscillometry is a process that is used to measure changes in pulsations in arteries, especially arteries of the extremities. Embodiments also generate a pressure-area curve and arterial volumetric blood flow waveforms over the entire transmural pressure range. Transmural pressure is pressure across the wall of a cardiac chamber or the wall of a blood vessel. Transmural pressure is calculated as intracavity pressure (*i.e.,* the pressure within the cardiac chamber or blood vessel) minus extracavity pressure (*i.e.,* the pressure outside the cardiac chamber or blood vessel).

Embodiments are used to measure reactive hyperemia, whereas other examples not covered by the present invention may be used to measure endothelial dysfunction, which is an early measure of a functional abnormality in the endothelium. The endothelium is the inner most layer of the arterial wall and is made up of a thin layer of flat endothelial cells. Endothelial dysfunction, or ED, is a well established response to cardiovascular risk factors and precedes the development of atherosclerosis (which leads to plaque development). When ED occurs, the magnitude of nitric oxide secretion is reduced and arterial vasodilation is also reduced. These changes lead to reduced reactive hyperemia that can be measured using the method and devices described herein.

ED is a predictor of overall vascular health. Accordingly, ED is an early indicator of cardiovascular disease. Indeed, ED has been shown to significantly and directly correlate to cardiovascular events such as myocardial infarction, stroke, sudden death, and heart failure. By measuring the degree of ED, embodiments described herein can be highly predictive of cardiovascular events and disease. Clinically, ED can predict the occurrence of de novo type II diabetes and the progression of metabolic syndrome to type II diabetes. ED is also associated with peripheral vascular disease and chronic renal failure, and has been shown to predict pre-eclampsia in pregnant women. Pre-eclampsia is a serious medical condition developing in late pregnancy for which there is no known cure. Pre-eclampsia is characterized by high blood pressure and proteinuria (protein in the urine). Pre-eclampsia may lead to blindness, kidney failure, liver failure, placental abruption, convulsions, and HELLP syndrome (a triad of Hemolytic anemia, Elevated Liver enzymes, Low Platelets). Pre-eclampsia occurs in as many as 10% of all pregnancies and can be fatal to both mother and child. ED has been shown to be an early warning sign for pre-eclampsia.

Additionally, interventional studies have shown a regression of ED with the treatment of risk factors through diet, exercise, weight loss, smoking cessation, diabetic management, and drugs such as statins and various lipid lowering medications. Consequently, embodiments described herein may also be used as a means to monitor progress and guide treatment and therapy in patients with ED-related diseases such as cardiovascular disease.

For example, there are two distinct populations in which measuring endothelial dysfunction (ED) is both clinically valuable and efficacious. One population consists of asymptomatic patients at risk for cardiovascular disease (CVD). The other population is patients with known CVD who are on medical therapy. In patients at risk for CVD, measuring ED can serve as an early sign of progressive heart disease. In at-risk patients taking medications for known CVD, measuring ED can also monitor progress and guide treatment.

In one example not according to the present invention, methods and devices described herein are used to monitor progress and guide treatment and therapy in patients who are taking statins. Currently, physicians prescribe statins but have no established method for measuring their effect on decreasing ED, an important component of how statin therapy works. Using reactive hyperemia as an indicator of ED, the methods and devices described herein could be used to measure the efficacy of statin drugs as well as risk factor modification (*i.e.,* weight loss, blood sugar control, smoking cessation etc) in improving ED. These methods involve measuring the reactive hyperemia and comparing the level of hyperemia in patients at different stages of the treatment. Where the efficacy of a medical treatment, such as statin treatment, is determined, the first measurement of reactive hyperemia is preferably a measurement taken prior to the initiation of the treatment, while the subsequent measurements are taken during the course of the statin treatment. The frequency of the measurement can be determined by the medical care provider. The medical care provider may further modify the treatment regimen based on the outcome the measurements.

The ability to measure accurate arterial flow waveforms using embodiments described herein may be beneficial in surgical, ambulatory and outpatient health care situations. Arterial flow waveform monitoring using embodiments described herein provides additional benefits beyond simple blood pressure monitoring. For example, a patient with hypovolemic shock would present the following physical manifestations in the earliest stage (referred to as the compensatory stage): increased heart rate, peripheral vasoconstriction and decreased cardiac output. An accurate arterial flow waveform measurement, such as provided by the embodiments described herein, can be obtained at any transmural pressure providing real-time, non-invasive measurement of cardiac output changes over time, in addition to constant accurate monitoring of heart rate and vascular tone.

Embodiments described herein measure reactive hyperemia (an increase in the quantity of blood flow to a body part resulting from the restoration of its temporarily blocked blood flow). Some embodiments make these measurements by combining oscillometry and segmental volume plethysmography fundamentals and applying non-linear equations for cuff compliance. Cuff compliance is the amount of volume change that takes place with the given pressure change of a blood pressure cuff. This relationship has demonstrated a non-linear relationship in previous studies. However, the non-linear relationship varies from cuff to cuff as well as depending on how the cuff is placed on the limb. An earlier patent, U.S. Patent No. 6,309,359 ("the '359 patent"), also combined oscillometry and segmental volume plethysmography fundamentals to make non-invasive determinations of peripheral arterial lumen area. However, the method and apparatus described in the '359 patent do not apply or use a non-linear, mathematical equation to generate a cuff compliance curve, instead simply using the cuff pressure that most closely corresponds to actual cuff pressure. The '359 patent method and apparatus also use different equipment, including requiring a high-frequency pump that operates at a frequency that is significantly higher than the arterial cycle frequency, which is typically in the range of 25-35 Hz. Moreover, each point of data used by the '359 patent is obtained as the cuff pressure descends, as opposed to during inflation (pressure increasing) and deflation (pressure decreasing).

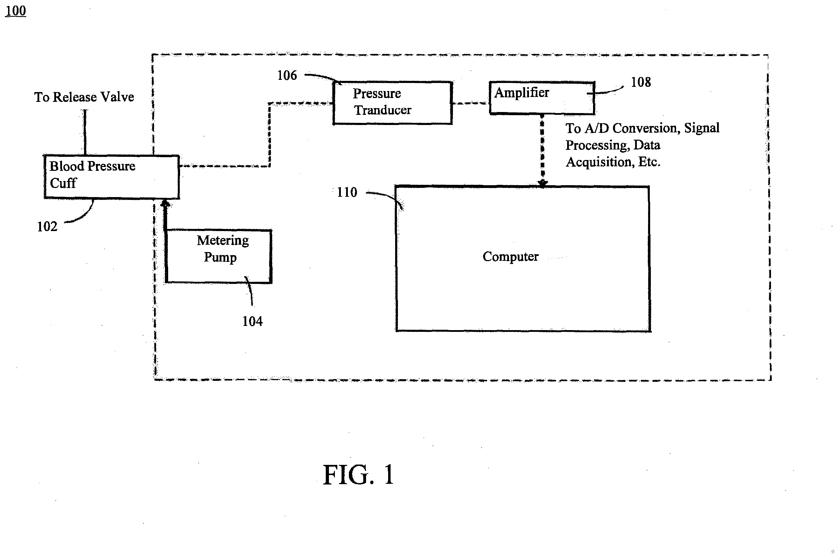

With reference now to FIG. 1, shown is a block diagram illustrating an embodiment of a system **100** for measuring arterial compliance. System **100** includes blood pressure cuff, meter, pump and hardware and/or software necessary for data acquisition described herein. Embodiment of system **100** includes a blood pressure cuff **102,** metering pump **104,** pressure transducer **106,** amplifier **108** and computer **110.** Blood pressure cuff **102** may be a standard blood pressure cuff **102** traditionally inflated to apply pressure to a limb so that blood pressure may be measured. The blood pressure cuff **102** is typically placed around the upper arm of the patient. However, the device could be used when placed around any portion of a limb for adults, children, or animals. Blood pressure cuff **102** size may be configured for the intended use in each case. Metering pump **104** includes a pump used to inflate blood pressure cuff **102** with a volume of air and a meter to measure inflation level of cuff (volume of air (*e.g.,* in liters/minute) injected into the cuff **102).** In one embodiment, the pump 104 is a low frequency pump. Pressure transducer **106** detects pulsatile pressure in the arteries of the limb by measuring the pressure in the cuff. The pressure transducer **106** generates a signal, indicative of the pulsatile pressure that is input into the amplifier **108.** Amplifier **108** amplifies the pulsatile pressure signal and inputs the amplified signal into the computer **110.**

The computer **110** may perform analog to digital (A/D) conversion of the amplified signal, as necessary, process the signal to acquire the necessary data, perform the methods, including calculating and applying the mathematical equations and generating the various curves, graphs and other displays, described herein. The computer **110** may be a general purpose computer with a processor(s) and memory that stores instructions (*e.g.,* as one or more computer programs) to perform these functions, or a special purpose computer so programmed. Accordingly, the software included on the computer **110** may include one or more data acquisition and mathematical programs and is capable of developing the non-linear mathematical functions used in the method, performing band-pass filtering and other filtering and signal processing as necessary, and performing mathematical transformations, *e.g.,* for area measurement under curves and numerical integration of arterial compliance curves for the development of pressure-area curves. Alternatively, instead of or in addition to computer **110,** system **100** may include circuitry to perform some of these functions, include a separate A/D converter, a band-pass filter and other specialized circuitry.

Components of the system **100,** such as the metering pump **104,** pressure transducer **106,** amplifier **108** and computer **110** may be housed within a single housing, as indicated by dashed lines in FIG. 1, connected to the blood pressure cuff **102.**

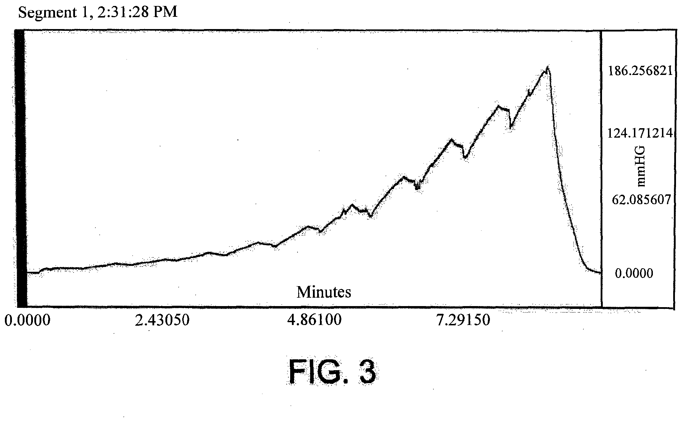

Embodiments of the method described herein obtain/acquire data during both inflation and deflation of the blood pressure cuff **102.** In embodiments, a known volume of air is injected into the blood pressure cuff **102** at each increment from 0 mm Hg to a pressure significantly higher than the patient's systolic blood pressure *(i.e.,* pressure that corresponds to the pressure in the arteries as the heart contracts and pumps blood into the arteries), yet not too uncomfortable for the patient (*e.g.,* approximately 180 mm Hg). Each pressure change (dP) is measured for each known volume change (dV) along the entire pressure ascent. FIG. 3 is a chart of data acquired during the inflation process.

In some embodiments, the above-described data (measured pressure change for each known volume change) obtained during the inflation is used to develop (dV/dP)$_{cuff}$ versus average cuff pressure curve (referred to as "the average cuff pressure curve"), where dV is change in volume, dP is change in pressure and (dV/dP)$_{cuff}$ is cuff compliance, which changes non-linearly with cuff pressure.

The data obtained above is plotted on the average cuff pressure curve. A non-linear regression is performed on this data, developing an equation where (dV/dP)$_{cuff}$ can be obtained at any cuff pressure. In one embodiment, the non-linear regression is performed using inverse polynomial second order functions. Successful coefficients of determination have been developed using such functions. In some embodiments, data points are obtained during deflation where Y = $(dV/dP)_{cuff}$ and x equals mean cuff pressure. From these points a nonlinear regression inverse second order polynomial equation can be generated.

In other embodiments, during deflation of the blood pressure cuff (the descent portion of the average cuff pressure curve), various additional data is obtained and additional functions are performed. In one embodiment, band pass filtering method (i.e., band pass filtering at various frequencies filters out other data so as to determine desired pressure data) may be used to obtain systolic blood pressure, diastolic blood pressure, and mean arterial blood pressure *(i.e.,* the average blood pressure during a single cardiac cycle *(i.e.,* over one cycle of a given arterial pressure waveform)) via oscillometry. In another embodiment, band pass filtering is used to provide the magnitude of pressure pulses that take place at each cuff pressure $(dP)_{artery}$.

Arterial compliance $(dV/dP)_{artery}$, which provides the measure of arterial smooth muscle activity and resulting endothelial dysfunction) at any transmural pressure can then be obtained via the following equation:

$$\left(\frac{dV}{dP}\right)_{artery} = \frac{(dP)_{artery} \, x \left(\frac{dV}{dP}\right)_{cuff}}{[Systolic - Diastolic]}$$

Equation 1. Arterial Compliance Calculation at Any Transmural Pressure

A curve is generated using Equation 1. The integration of the curve obtained from Equation 1 results in a pressure-area (P-A) curve. (Note: all volume measurements can be converted to area measurements if volume is divided by effective cuff length). All compliance measurements can be normalized by using Equation 2 below, where all volume measurements can be converted to area by once again dividing by effective cuff length.

$$C = \frac{\left(dV/dP\right)_{artery}}{V_0}$$

Equation 2. Normalized Arterial Compliance Equation

$V_0$ is base volume of patient. By dividing the arterial compliance by the patient base volume, the arterial compliance is normalized. The process described above may be used to perform a variety of measurements.

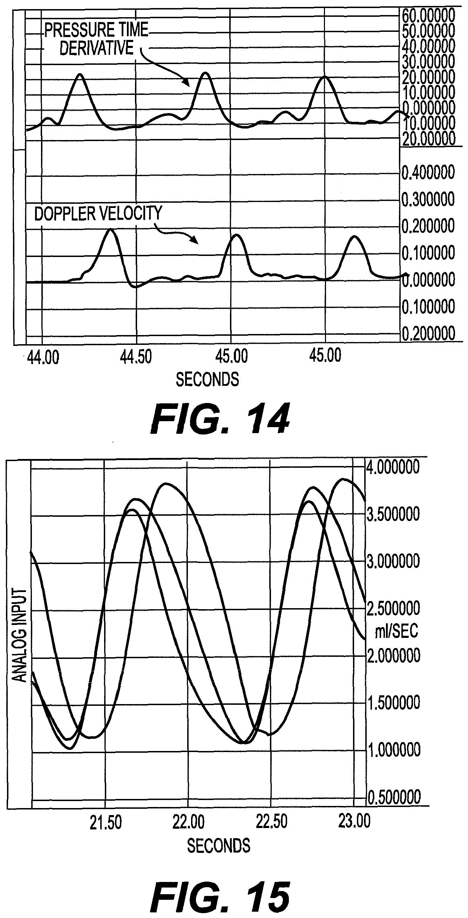

In one embodiment, the process is used for the development of an accurate arterial flow waveform by obtaining values for $(dV/dP)_{artery}$ at any transmural pressure, taking derivative of the original pressure descent waveform to obtain a waveform that is $(dP/dt)_{artery}$, and multiplying $(dP/dt)_{artery}$ by $(dV/dP)_{artery}$ to obtain an accurate flow waveform. The result is an accurate arterial flow waveform $(dV/dt)_{artery}$. FIG. 14 shows an exemplary pressure-time derivative and Doppler velocity waveforms and FIG. 15 shows exemplary flow waveforms calculated from pressure-time derivatives

In another example not according to the present invention, the process is used for the measurement of endothelial dysfunction by obtaining a baseline arterial compliance curve and pressure-area curve as discussed above, holding the blood pressure cuff above a patient's systolic pressure for a given period of time (inducing hyperemia), obtain a second (*i.e.,* hyperemic) compliance curve and pressure-area curve, and calculating the difference between the baseline curve and the hyperemic curve. The difference between the two curves represents the degree of endothelial dysfunction.

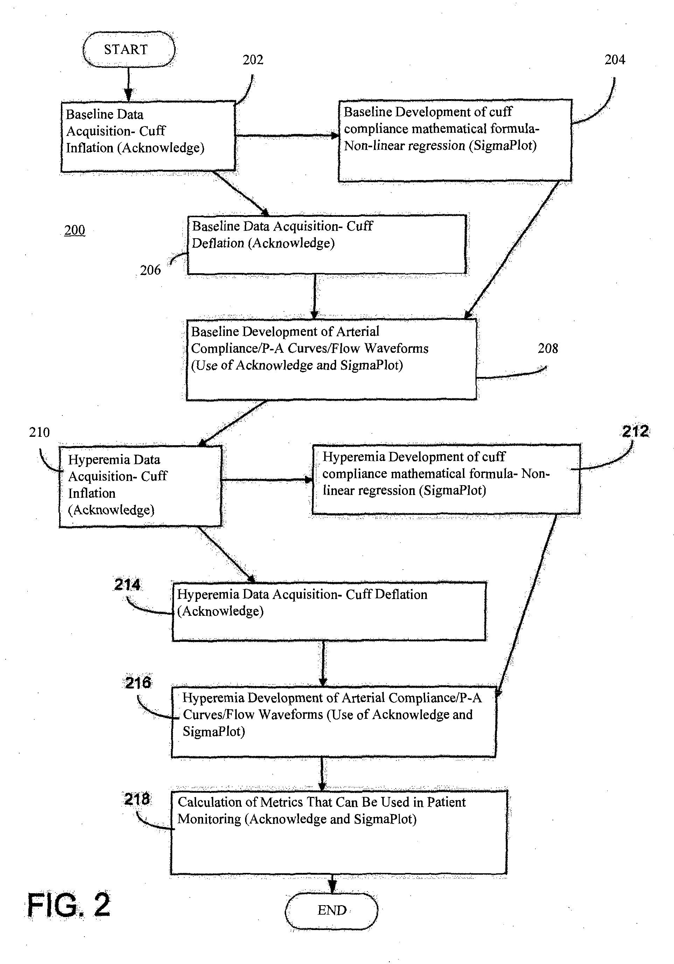

With reference now to FIG. 2, shown is a flowchart illustrating a method **200** for measuring arterial compliance. Baseline data is acquired during cuff inflation, block **202**. See FIG. 3 for a graph of baseline data acquired during cuff inflation. The baseline data acquisition **202** may include using the metering pump **104** and pressure transducer **106** during cuff **102** inflation to measure air volume injection into the pump and cuff pressure, respectively. The baseline data acquisition **202** may further include placing the cuff **102** about a peripheral limb of a patient or subject and starting the cuff at 0 mm Hg. A known volume of air may be injected into the cuff **102** while recording cuff pressure change. The baseline data acquisition **202** may be accomplished, *e.g.,* using data acquisition software program running on computer **110**. The data acquisition program may receive input from pressure transducer **106** and/or metering pump **104** *(e.g.,* that has been converted to digital via A/D converter and/or otherwise signal processed). A variety of data acquisition programs may be used, such as, for example, Acknowledge™. The baseline data acquisition **202** obtains the cuff compliance at given average cuff pressure, where average cuff pressure equals the cuff pressure after a known volume of air injection plus the cuff pressure before the known volume of air injection, divided by two (2). As described above, the volumetric inflation of the cuff is continued up to a pressure significantly higher than the subject systolic pressure yet not too uncomfortable for the subject (*e.g.*, approximately 180 mm Hg).

Baseline cuff compliance equation/formula is generated, block **204.** As indicated above, this may be achieved using non-linear regression. In embodiments, the baseline cuff compliance formula generation can begin as soon as cuff inflation has been completed. A mathematical equation representing cuff compliance may be developed by (i) plotting average cuff pressure (x) versus cuff compliance (y) and performing a non-linear regression as stated above. The baseline cuff compliance equation/formula may be generated **204** using a mathematical software program running on computer **110.** The mathematical program may receive input from the data acquisition program and may perform, *e.g.,* non-linear regression using, *e.g.,* an inverse polynomial second order function(s). A variety of data acquisition programs may be used, such as, for example, SigmaPlot™.

With continued reference to FIG. 2, additional baseline data is acquired during cuff deflation as described above, block **206.** Additional baseline data acquisition **206** may be performed, e.g., using data acquisition program. After this additional baseline data acquisition **206** is performed, band-pass filtering and development of arterial compliance curves, P-A curves and arterial flow waveforms are performed, block **208.**

To develop baseline curves and waveforms, the pressure in the cuff is immediately released after the cuff pressure has gone above the patient systolic pressure and the measurements described above are taken. In other words, cuff pressure is raised so that it is above the patient systolic pressure and then immediately released. To develop reactive hyperemia curves and waveforms, the cuff pressure is held for a period of time sufficient to trigger an endothelial reaction and the measurements described above are taken. In embodiments described herein, the period of time is the time that is necessary to achieve total relaxation of the patient's smooth muscle tissue. Relaxation of the smooth muscle tissue is typically necessary to achieve accurate reactive hyperemic measurements. In one embodiment, the cuff pressure is held for a period of 1-10 minutes. In another embodiment, the cuff pressure is held for a period of 2-5 minutes. In yet another embodiment, the cuff pressure is held for about 5 minutes. The longer the cuff pressure can be held, the more assured the tester will be that the patient's smooth muscles have been relaxed and accurate reactive hyperemic measurements will be taken. The usual limiter on holding the cuff pressure is patient comfort; the longer the cuff pressure is held the more uncomfortable and, eventually, painful the procedure becomes. Elderly, ill or weakened patients tend to be able to withstand less time than younger, healthy patients.

After the baseline data has been generated, as described above, hyperemia data may be generated by basically repeating the above steps after a brief period of patient hyperemia. Hyperemia data is acquired during inflation of the cuff, block **210.** The hyperemia data may be acquired by again starting the cuff at 0 mm Hg and inflating the cuff as described above. Acquisition **210** may include using the metering pump **104** and pressure transducer **106** during cuff **102** inflation to measure air volume injection into the pump and cuff pressure, respectively. Again, a known volume of air may be injected into the cuff **102** while recording cuff pressure change.

With continuing reference to FIG. 2, hyperemia cuff compliance mathematical equation is generated, block **212.** Again, the hyperemia cuff compliance mathematical equation may be generated as described above. Additional hyperemia data is acquired during cuff deflation, block **214.** After this additional data is acquired, band-pass filtering and development of arterial compliance curves, P-A curves and arterial flow waveforms for hyperemia are performed, block **216.** Metrics to detect conditions or monitor the patient are calculated, block **218.** The metrics may include, *e.g.,* (a) comparing various curves of baseline data and hyperemic data, (b) calculating the differences between the curves as an area between the curves, and, *e.g.,* (c) determining a level of ED (or, *e.g.*, the presence of pre-eclampsia or other diseases, etc.) based on the calculated area. For example, such metrics may provide outputs indicating, for example, the presence of ED. Alternative (c) is not part of the present invention.

In one embodiment, the change between arterial compliance normal (baseline) curve, generated in **208,** and the arterial compliance hyperemia curve, generated in **216,** is calculated at a given transmural pressure.

In another embodiment, the change between arterial compliance normal (baseline) curve, generated in **208,** and the arterial compliance hyperemia curve, generated in **216,** can be calculated across a range of transmural pressures.

In another embodiment, the change between P-A normal (baseline) curve, generated in **208,** and the P-A reactive hyperemia curve, generated at **216,** is calculated at a given transmural pressure.

In another embodiment, the change between P-A normal (baseline) curve, generated in **208,** and the P-A reactive hyperemia curve, generated at **216,** is calculated across a range of transmural pressures.

In another embodiment, arterial flow waveforms (at any given transmural pressure or range of transmural pressures) for normal (baseline) curves and/or reactive hyperemia curves are calculated and compared.

In another embodiment, average flow waveforms (at any given transmural pressure or range of transmural pressures) for normal (baseline) curves and/or reactive hyperemia curves are calculated and compared.

In another embodiment, the computer **110** contains means for calculating an arterial compliance curve and a P-A curve during the inflation and deflation process of the cuff, means for calculating difference between areas under a first arterial compliance curve and a second arterial compliance curve and difference between areas under a first pressure-area (P-A) curve and a second P-A curve, and means for band pass filtering data.

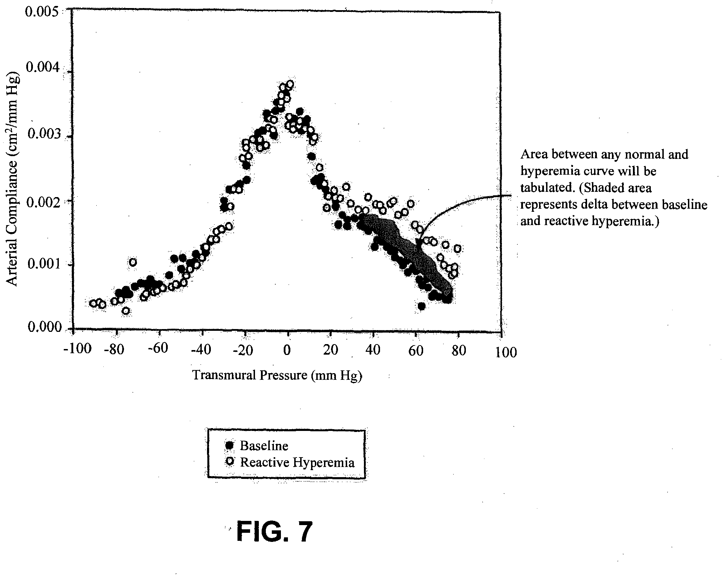

Many of these metrics are significant in that they have never been performed over a range of transmural pressures. Other devices that perform similar metrics only do so for single points of data. Conducting these metrics over a range of transmural pressures allows more data to be compared and more information gathered from the results. The above metrics enable results to be compared over a range of pressures. More importantly, comparing the above curves of data enables the differences between the curves to be calculated as an area (see FIGS. 7-8). The lesser the area between the curves, the higher degree of ED; in other words, there is an inverse relationship between the area and the amount of ED. In certain examples not according to the present invention, a scoring system is developed in which certain amounts of area (*e.g.*, a range of area), calculated from the differences between the curves, correspond to certain levels of ED.

With reference to FIG. 3, shown is a graph of sample data acquisition during cuff inflation and deflation. The graph shows cuff pressure over time. By noting the time, the change in cuff pressure over a given time can be determined and compared to the change of cuff injection volume (not shown here) over the same time. Using this data, cuff compliance and average cuff pressure can be calculated.

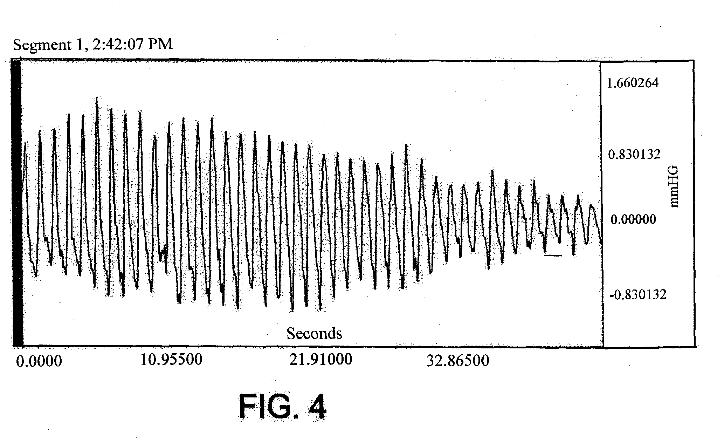

With reference to FIG. 4, shown is a graph of sample data obtained during descent (deflation of cuff) that has been band pass filtered to provide arterial pressure pulses. The band pass filtering removes other pressure data received from the pressure transducer to leave only, in this example, the arterial pressure pulse data. In this example, the band pass filtering may be performed at 0.5 to 5.0 Hertz.

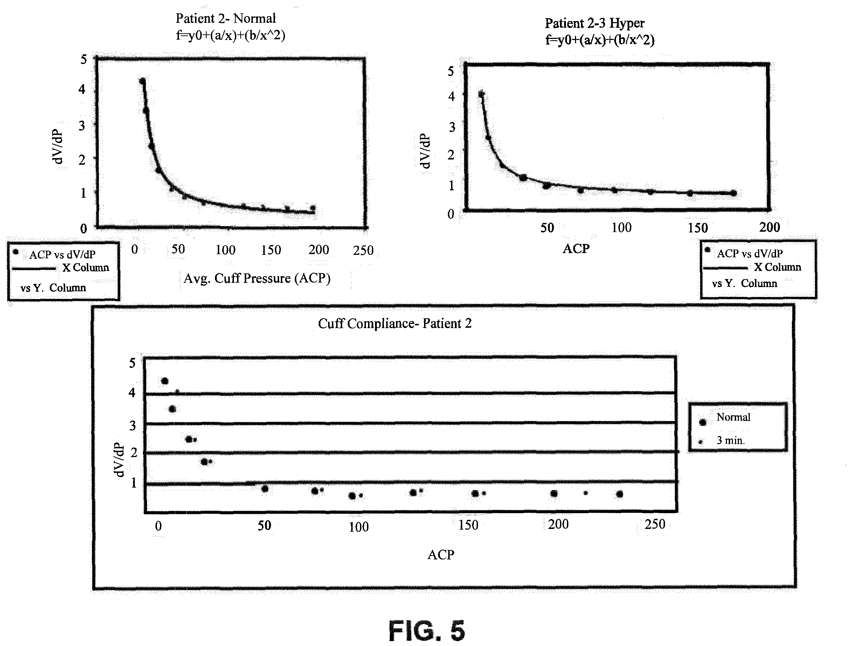

With reference to FIG. 5, shown are example cuff compliance curves generated during pressure ascent (inflation of the cuff). The cuff compliance curves may be generated as described above.

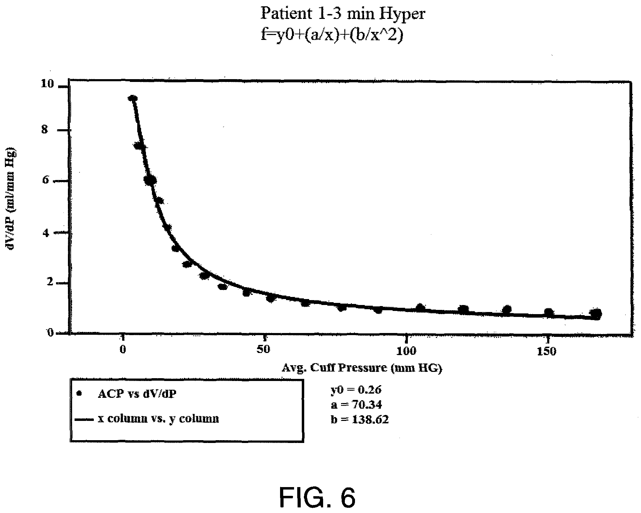

With reference to FIG. 6, shown is an example cuff compliance curve generated during pressure ascent using coefficients generated using non-linear regression described above. For example, inverse polynomial second order functions may be used.

The following is sample equation output where non-linear equation for cuff compliance was developed as described above. Specifically, the information below is a sample output from a non-linear regression (using an inverse polynomial second order function) performed to develop the cuff compliance curve.

Sample Output from Non-Linear Regression Used to Develop Cuff Compliance Curve

**Nonlinear Regression**

**Equation: Polynomial, Inverse Second Order**

$$f = y0 + (a/x) + (b/x^2)$$

| R | Rsqr | Adj Rsqr | Standard Error of Estimate | | |
|---|---|---|---|---|---|
| 0.9947 | 0.9895 | 0.9883 | 0.2858 | | |
| | Coefficient | Std. Error | t | P | VIF |
| y0 | 0.2598 | 0.1091 | 2.3811 | 0.0285 | 3.0614 |
| a | 70.3371 | 3.3041 | 21.2880 | <0.0001 | 17.8907< |
| b | -138.6316 | 15.6635 | -8.8506 | <0.0001 | 12.2896< |

**Analysis of Variance:**

Uncorrected for the mean of the observations:

| | DF | SS | MS |
|---|---|---|---|
| Regression | 3 | 349.7290 | 116.5763 |
| Residual | 18 | 1.4705 | 0.0817 |
| Total | 21 | 351.1995 | 16.7238 |

Corrected for the mean of the observations:

|            | DF | SS       | MS      | F        | P       |
|------------|----|----------|---------|----------|---------|
| Regression | 2  | 138.7197 | 69.3598 | 849.0321 | <0.0001 |
| Residual   | 18 | 1.4705   | 0.0817  |          |         |
| Total      | 20 | 140.1902 | 7.0095  |          |         |

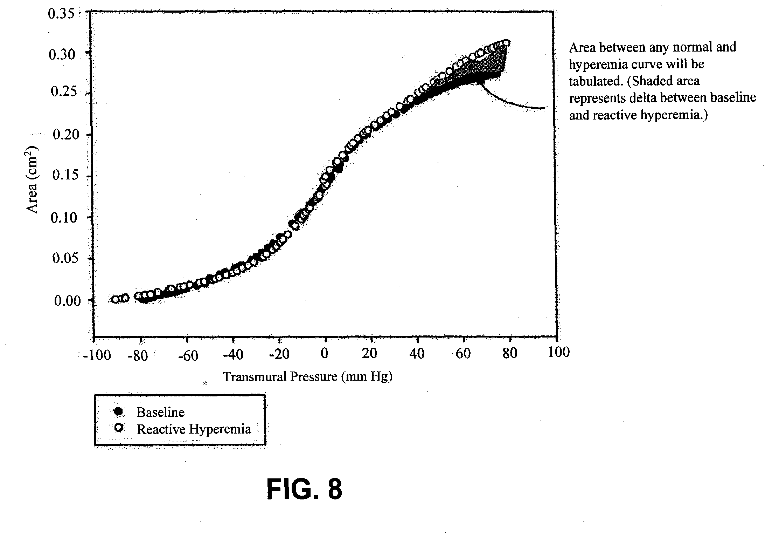

The resulting arterial compliance versus transmural pressure curves (arterial compliance curve) can be shown with the unit of ml/ mm Hg or ml for the Y-axis, or with the unit of $cm^2$/ mm Hg or $cm^2$ for the Y-axis (see FIGS. 7 and 8), which is derived by dividing the volume in ml/ mm Hg or ml with the effective cuff length. In some embodiments, the final arterial compliance curves are shown with the unit of ml/ mm Hg or ml for the Y-axis. In other embodiments, the final arterial compliance curves are shown with the unit of $cm^2$/ mm Hg or $cm^2$ for the Y-axis.

The differential between normal area measurements and hyperemia measurements provides a quantitative measure of endothelial dysfunction. For example, FIG. 7 is a graph illustrating baseline and hyperemia arterial compliance curves. The differential/change between the two curves is indicated by the shaded area, which indicates the presence of endothelial dysfunction. The size of this shaded area is inversely proportional to the magnitude of endothelial dysfunction or the risk/presence of ED-related diseases (*e.g.*, the risk/presence of pre-eclampsia).

In one example not according to the present invention, a differential percentage (*i.e.,* the area between the baseline arterial compliance curve and the hyperemia arterial compliance curve divided by the area under the baseline arterial compliance curve) of 10% or less indicates ED or the risk/presence of an ED-related disease. In another example not according to the present invention, a differential percentage less than 7% indicates ED or the risk/presence of an ED-related disease. In another embodiment, differential percentage less than 4.5% indicates ED or the risk/presence of an ED-related disease. In summary, the lesser differential area between the baseline arterial compliance curve and the hyperemia arterial compliance curve, the greater the indication of ED or the risk/presence of an ED-related disease. In other examples not according to the present invention, different thresholds are given to patient populations of different age, sex, race or geographic location. In another example not according to the present invention, a scoring chart for ED based on this area, are developed in which different levels of ED correspond to different sizes of the shaded area.

In a preferred embodiment, the differential in the arterial compliance curves is determined in the portion from zero transmural pressure to the maximum transmural pressure.

In certain embodiment, a single point from a patient's normal (baseline) arterial compliance curve is compared to a single point from the patient's hyperemic arterial compliance curve at the same transmural pressure value. In a preferred embodiment, the single point comparison of the arterial compliance curves is made in the portion from zero transmural pressure to the maximum transmural pressure.

Likewise, FIG. 8 is a graph illustrating baseline and hyperemia P-A curves. The differential/change between the two curves is indicated by the shaded area, which is inversely proportional to the presence of endothelial dysfunction. The area of this shaded area is inversely proportional to the magnitude of endothelial dysfunction or other disease (*e.g.*, the presence of pre-eclampsia). In one example not according to the present invention, a differential percentage (*i.e.,* the area between the baseline P-A curve and the hyperemia P-A curve divided by the area under the baseline P-A curve) of 10% or less indicates ED or the risk/presence of an ED-related disease. In another example not according to the present invention, a differential percentage less than 7% indicates ED or the risk/presence of an ED-related disease. In another example not according to the present invention, differential percentage less than 4.5% indicates ED or the risk/presence of an ED-related disease. In summary, the lesser differential area between the baseline P-A curve and the hyperemia P-A curve, the greater the indication of ED or the risk/presence of an ED-related disease.

In another example not according to the present invention, different thresholds are given to patient populations of different age, sex, race or geographic location In another example not according to the present invention, a scoring chart for ED based on this area, are developed in which different levels of ED correspond to different sizes of the shaded area.

In a preferred embodiment, the differential in the P-A curves is determined in the portion from zero transmural pressure to the maximum transmural pressure.

In certain embodiment, a single point from a patient's normal (baseline) P-A curve is compared to a single point from the patient's hyperemic P-A curve at the same transmural pressure value. In a preferred embodiment, the single point comparison of the arterial compliance curves is made in the portion from zero transmural pressure to the maximum transmural pressure.

In another embodiments, the actual flow waveform at any given transmural pressure is calculated. The actual flow waveform may be calculated at any transmural pressure where blood flow is permitted by cuff pressure.

In other examples not according to the present invention, the level of ED is used as an indicator for a diseased condition or as an indicator for the risk of a diseased condition. Such diseased conditions include, but are not limited to, pre-eclampsia, hypertension, atherosclerosis, cardiovascular disease (including coronary artery disease and stroke), atrial fibrillation, congestive heart failure, peripheral vascular disease, septic shock, heat stress, hypercholesterolemia, type I and II diabetes, erectile dysfunction, rheumatic arthritis, HIV, and liver disease (cirrhosis, hepatitis B and C, non-alcoholic steatohepatitis, fatty liver disease) pre-eclampsia, all forms of dementia and psychological illness, any and all illness related to localized or systemic inflammation and obesity.

In other examples not according to the present invention, the pressure/volume measurements taken during the segment cuff plethysmography are used for purposes other than measuring ED. For example, the pressure/volume measurements can be used to monitor cardiac function by taking the derivative of pressure waveform and mathematically superimposing all points with the nonlinear cuff compliance relationship to generate a calibrated flow waveform.

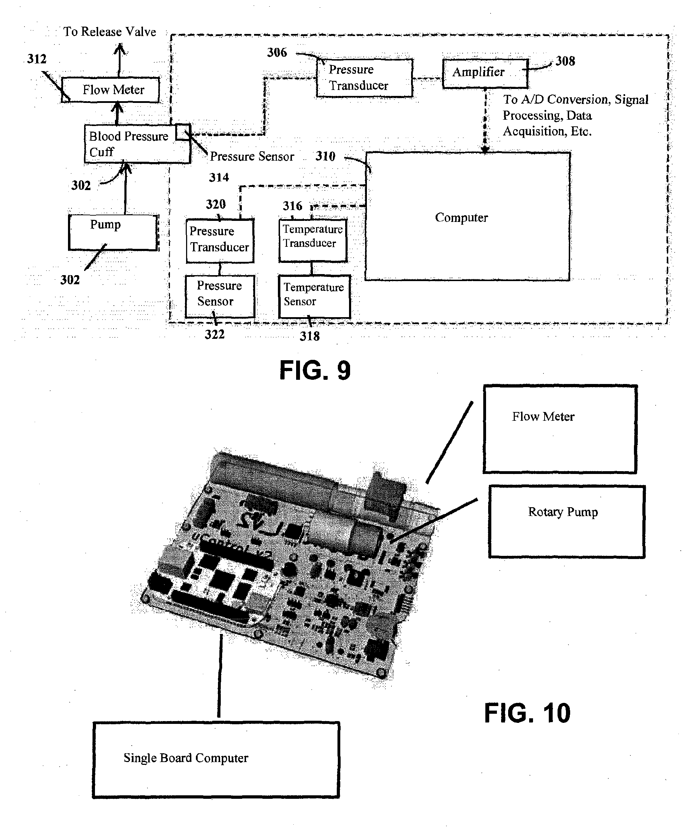

With reference now to FIG. 9, shown is a block diagram illustrating another embodiment of a system **300** for measuring arterial compliance. In some embodiments, the system **300** includes a blood pressure cuff **302** having an air inlet and an air outlet, a pump **304** connected to the air inlet of the cuff **302,** a pressure transducer **306,** an amplifier **308,** a computer **310** and a flowmeter **312** connected to the air outlet of the cuff **302.** In one embodiment, the pump **304** is a rolling pump that inflates the blood pressure cuff **302** with a volume of air. In one embodiment, the pump **304** is a low frequency pump that operates at a frequency that is lower than the arterial cycle frequency. In other embodiments, the pump **304** is a low frequency pump that operates at a frequency below 10 Hertz, below 5 Hertz, below 2 Hertz, below 1 Hertz, below 0.5 Hertz, below 0.2 Hertz, below 0.1 Hertz, below 0.05 Hertz, below 0.02 Hertz, or below 0.01 Hertz. In some embodiments, the pump **304** also contains a meter to measure the volume of air injected into the cuff **302** during cuff inflation. The flowmeter **302** measures the volume of air released from the cuff **302** during cuff deflation. The pressure transducer **306** detects pulsatile pressure in the arteries of the limb by measuring the pressure in the cuff **302** through a pressure sensor **314.** The pressure transducer **306** generates a signal, indicative of the pulsatile pressure that is input into the amplifier **308.** Amplifier **308** amplifies the pulsatile pressure signal and inputs the amplified signal into the computer **310.** In some embodiments, the system 300 further comprises a temperature sensor **318** and a pressure sensor **322** that measure the ambient temperature and pressure, respectively, as well as a temperature transducer **316** and a pressure transducer **320** that generate the ambient temperature signal and ambient pressure signal for processing at the computer **310.** The purpose of the ambient pressure/temperature measurement is to provide a correction factor for volumetric measurement taking place at the flowmeter **312** using the ideal gas law principle PV = nRT

With reference now to **FIG. 10,** shown is a schematic illustrating an embodiment of an integrated printed circuit board (PCB) **350** for the system **300.** The integrated PCB **350** comprises the pump **304,** the flowmeter **312,** the pressure transducer **306,** the amplifier **308,** and the computer **310.**

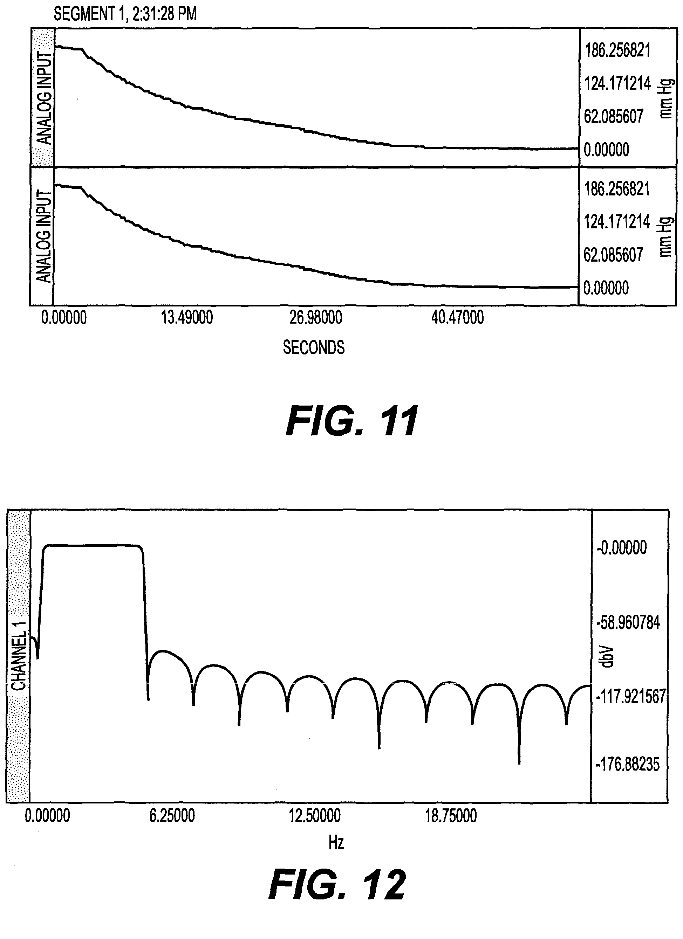

In some embodiments, all data acquisition takes place during cuff deflation and arterial compliance curve, P-V curve and P-A curve are all generated based only on data collected during the cuff deflation phase. **FIG. 11** shows an exemplary cuff deflation curve. During the deflation phase, the cuff pressure decreased from about 200 mmHg to about 0 mmHg. The following information is obtained

    i. Mean Cuff Pressure (MCP)
    ii. decremental Volume Change (dV)
    iii. decremental Pressure Change (dP)

The volumetric decrements (dV) is directly measured by the flowmeter **312.** The decremental pressure change (dP) corresponding with each volumetric decrement (dV) is also directly measured by the pressure transducer **306** through the pressure sensor **314** on the cuff **302.** The Cuff compliance (CC) is calculated for each MCP.

$$CC = (dV/dP)_{cuff}$$

A cuff compliance curve is then generated based on measured pressure change for each measured volume change obtained during the deflation. During deflation, the actual volume measurements provide increased accuracy in the formulation of the cuff compliance values as the pressure descends at all cuff pressures. This leads to increased accuracy of the arterial compliance term. The benefit in this accuracy is that clinicians now have temporal data that allows them to compare measurement over any time period

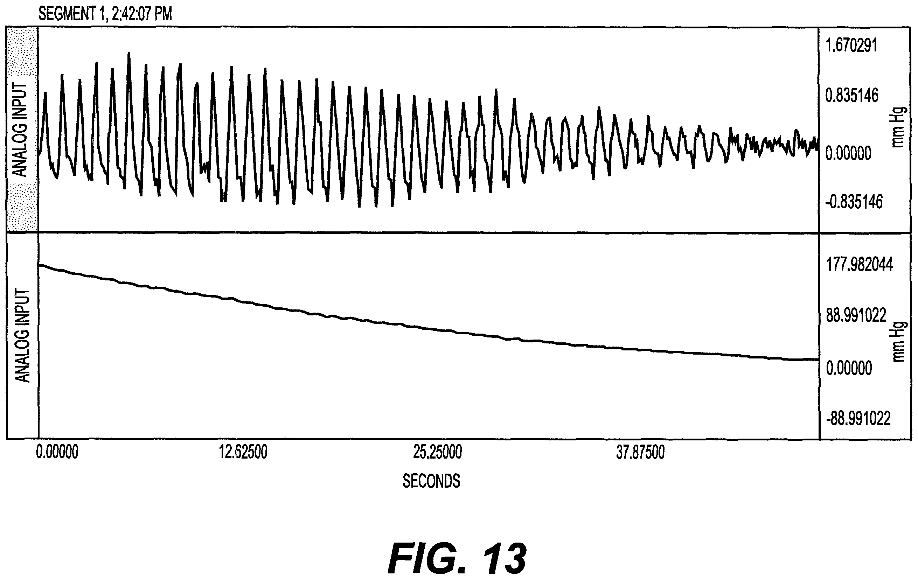

Next, a band pass filter (0.5-5.0 Hertz) is applied to the cuff pressure curve data. **FIG. 12** shows an exemplary band pass filter frequency response output. **FIG. 13** shows band pass filter (0.5 - 5.0 Hertz) of cuff descent data (top panel) and standard cuff descent data (bottom panel). In one embodiment, an additional DC low pass filter (0 - 0.5 Hz) is used to obtain the flowrate as air leaves the cuff. This is used to obtain the actual volume changes. The mean arterial pressure (MAP), Systolic Blood Pressure (SBP) and Diastolic Blood Pressure (DBP) are calculated using the following rules:

    (a) MAP corresponds with the largest peak-to-peak measurement. Record the magnitude;
    (b) SBP corresponds with approximately 55% of the magnitude of the largest peak-to-peak measurement. Find the peak-to-peak value that corresponds most closely with this value (at a cuff pressure greater than MAP) and find the cuff pressure. This cuff pressure is equivalent to SBP; and

(c) DBP corresponds with approximately 85% of the magnitude of the largest peak-to-peak measurement. Find the peak-to-peak value that corresponds most closely with this value (at a cuff pressure less than MAP) and find the cuff pressure. This cuff pressure is equivalent to DBP.

The arterial compliance curve ($(dV/dP)_{artery}$ v. transmural pressure) is then generated via the following calculation:

i. $(dV/dP)_{artery} = [(dV/dP)_{cuff} \times dPc_{uff}]/dP_{artery}$
ii. $dP_{artery} = SBP - DBP$
iii. Transmural pressure = MAP - Cuff Pressure.

A pressure-volume (P-V) curve and pressure-area (P-A) curve can be generated via integration of the arterial compliance curve (designated as baseline arterial compliance, P-V and P-A curves). The procedure is then repeated during the second segmental plethysmography, where the cuff pressure is held for a period of 1-10 minutes, 2-8 minutes, 2-5 minutes or about 5 minutes, to produce the arterial compliance, pressure-volume (P-V) and pressure-area (P-A) curves (designated as hyperemia arterial compliance, P-V and P-A curves). In some examples not according to the present invention, the area between the baseline and hyperemia arterial compliance curves is calculated and used for the evaluation of ED. In some examples, only the area that is within the transmural pressure range of 0-120 mmHg, 0-100 mmHg, 0-80 mmHg, 20-120 mmHg, 20-100 mmHg, or 20-80 mmHg is calculated.

In some examples not according to the present invention, the area between the baseline and hyperemia P-A curves is calculated and used for the evaluation of ED. In some embodiments, only the area that is within the transmural pressure range of 0-120 mmHg, 0-100 mmHg, 0-80 mmHg, 20-120 mmHg, 20-100 mmHg, or 20-80 mmHg is calculated.

The embodiments described herein enable a physician, for example, to both measure and monitor reactive hyperemia, and, outside the scope of the present invention, ED, ED-related diseases, cardiovascular conditions and the efficacy of various forms of treatment. Key metrics obtained include actual measurements of peripheral arterial flow, arterial compliance, and arterial area across the entire arterial transmural pressure range.

A significant benefit of the embodiments described herein is the early diagnosis of ED-related diseases, which, however, is not part of the present invention. A physician, for example, is able to gain valuable information from the reactive hyperemic measurement. Device embodiments provide benefits to clinicians in providing a simple method to diagnose patients that are currently classified as asymptomatic, as well as quantify the efficacy of current and novel treatments in patients already diagnosed with disease. Another significant benefit of the embodiments described herein is the ability to monitor the effectiveness of treatments for ED, which, however, is not part of the present invention.

The terms and descriptions used herein are set forth by way of illustration only and are not meant as limitations. Those skilled in the art will recognize that many variations are possible within the scope of the invention as defined in the following claims, in which all terms are to be understood in their broadest possible sense unless otherwise indicated.

**Claims**

1. A method for measuring reactive hyperemia in a subject, comprising:

performing a first segmental cuff plethysmography having an inflation phase and a deflation phase;
generating a baseline arterial compliance curve and/or a baseline pressure-area (P-A) curve on a portion of the body of the subject, wherein the cuff pressure is increased to a first peak cuff pressure during the inflation phase and immediately reduced from the first peak cuff pressure during the deflation phase;
performing a second segmental cuff plethysmography having an inflation phase, a holding phase, and a deflation phase;
generating a hyperemic arterial compliance curve and/or a hyperemic P-A curve, wherein the cuff pressure is increased to a second peak level during the inflation phase, maintained at the second peak cuff pressure for a predetermined period of time during the holding phase, and then reduced from the second peak cuff pressure during the deflation phase;
calculating the difference between the baseline arterial compliance curve and the hyperemic arterial compliance curve as an area between the arterial compliance curves, and/or the difference between the baseline P-A curve and the hyperemic P-A curve as an area between the PA curves; and
measuring a level of reactive hyperemia based on the area between the arterial compliance curves and/or the area between the P-A curves,

wherein a first cuff compliance curve is generated during the first segmental cuff plethysmography based only on data collected during the deflation phase of the first segmental cuff plethysmography and a second cuff compliance curve is generated during the second segmental cuff plethysmography based only on data collected during the deflation phase of the second segmental cuff plethysmography and wherein said first and second cuff compliance curves are generated using a flowmeter (302) that directly measures the volume change in the cuff during the deflation phase.

2. The method of Claim 1, wherein the first peak cuff pressure and the second peak cuff pressure are in the range of 150-180 mmHg.

3. The method of Claim 1 or 2, wherein the predetermined period of time is 2-10 minutes.

4. The method of Claim 1, wherein the calculating step calculates the difference between the baseline arterial compliance curve and the hyperemic arterial compliance curve as an area between the arterial compliance curves within a transmural pressure range of 0-100 mmHg, and wherein the calculating step calculates the difference between the baseline P-A curve and the hyperemic P-A curve as an area between the P-A curves within a transmural pressure range of 0-100 mmHg.

5. The method of Claim 4, wherein the calculating step calculates the difference between the baseline arterial compliance curve and the hyperemic arterial compliance curve as an area between the arterial compliance curves within a transmural pressure range of 20-80 mmHg, and wherein the calculating step calculates the difference between the baseline P-A curve and the hyperemic P-A curve as an area between the P-A curves within a transmural pressure range of 20-80 mmHg.

6. The method of Claim 1, wherein the generation of said baseline arterial compliance curve and/or a baseline pressure-area (P-A) curve comprising applying a band pass filter of 0.5-5.0 Hertz to cuff pressure data collected during the deflation phase of said first segmental cuff plethysmography and wherein the generation of said hyperemia arterial compliance curve and/or hyperemia pressure-area (P-A) curve comprising applying a band pass filter of 0.5-5.0 Hertz to cuff pressure data collected during the deflation phase of said second segmental cuff plethysmography.

7. The method of Claim 6, further comprising the step of applying an additional DC low pass filter (0 - 0.5 Hz) to data generated by the pressure transducer during the deflation phase of said first and second segmental cuff plethysmography to generate flowrate data.

8. An apparatus comprising:

   an inflatable cuff having an inlet and an outlet;
   a pump (304) connected to said inlet of said cuff for inflating the cuff;
   a flowmeter connected to said outlet of said cuff;
   a pressure transducer (306) for measuring the pressure inside the cuff; and
   a computer (310) configured to:

   generate a cuff compliance curve by directly measuring volume change in said cuff with said flowmeter and pressure change inside the cuff with said pressure transducer during a deflation process of the cuff;
   calculate an arterial compliance curve, which includes a first arterial compliance curve and a second arterial compliance curve, and a pressure area (P-A) curve, which includes a first P-A curve and a second P-A curve, only during the deflation process of the cuff; and
   calculate a difference between areas under the first arterial compliance curve and the second arterial compliance curve and a difference between areas under the first P-A curve and the second P-A curve.

9. The apparatus of Claim 8, wherein said pump is a low-frequency pump having an operation frequency of 10 Hertz or lower.

10. The apparatus of Claim 8, wherein said pump is a low-frequency pump having an operation frequency of 2 Hertz or lower.

**Patentansprüche**

1. Verfahren zur Messung von reaktiver Hyperämie bei einem Subjekt, das Folgendes umfasst:

   Durchführen einer ersten segmentären Plethysmographie mit Manschette, die eine Aufblasphase und eine Entleerungsphase hat;

   Erzeugen einer Grundlinienkurve der arteriellen Compliance und/oder einer Druck-Fläche (P-A)-Grundlinienkurve an einem Teil des Körpers des Subjekts, wobei der Manschettendruck auf einen Manschettendruck eines ersten Höchstwerts während der Aufblasphase erhöht wird und sofort von dem Manschettendruck des ersten Höchstwerts während der Entleerungsphase reduziert wird;

   Durchführen einer zweiten segmentären Plethysmographie mit Manschette, die eine Aufblasphase, eine Haltephase und eine Entleerungsphase hat;

   Erzeugen einer hyperämischen Kurve der arteriellen Compliance und/oder einer hyperämischen P-A-Kurve, wobei der Manschettendruck auf eine Höhe eines zweiten Höchstwerts während der Aufblasphase erhöht wird, bei dem Manschettendruck des zweiten Höchstwerts für eine vorgegebene Zeitdauer während der Haltephase gehalten wird und dann von dem Manschettendruck des zweiten Höchstwerts während der Entleerungsphase reduziert wird;

   Berechnen des Unterschieds zwischen der Grundlinienkurve der arteriellen Compliance und der hyperämischen Kurve der arteriellen Compliance als eine Fläche zwischen den Kurven der arteriellen Compliance und/oder des Unterschieds zwischen der P-A-Grundlinienkurve und der hyperämischen P-A-Kurve als eine Fläche zwischen den P-A-Kurven; und

   Messen einer Höhe von reaktiver Hyperämie basierend auf der Fläche zwischen den Kurven der arteriellen Compliance und/oder der Fläche zwischen den P-A-Kurven, wobei eine erste Compliance-Kurve mit Manschette während der ersten segmentären Plethysmographie mit Manschette basierend nur auf Daten erzeugt wird, die während der Entleerungsphase der ersten segmentären Plethysmographie mit Manschette gesammelt werden, und eine zweite Compliance-Kurve mit Manschette während der zweiten segmentären Plethysmographie mit Manschette basierend nur auf Daten erzeugt wird, die während der Entleerungsphase der zweiten segmentären Plethysmographie mit Manschette gesammelt werden, und wobei die erste und zweite Compliance-Kurve mit Manschette unter Verwendung eines Durchflussmessers (302) erzeugt werden, der die Volumenänderung in der Manschette während der Entleerungsphase direkt misst.

2. Verfahren nach Anspruch 1, wobei der Manschettendruck des ersten Höchstwerts und der Manschettendruck des zweiten Höchstwerts in dem Bereich von 150-180 mmHg liegen.

3. Verfahren nach Anspruch 1 oder 2, wobei die vorgegebene Zeitdauer 2-10 Minuten beträgt.

4. Verfahren nach Anspruch 1, wobei der Berechnungsschritt den Unterschied zwischen der Grundlinienkurve der arteriellen Compliance und der hyperämischen Kurve der arteriellen Compliance als eine Fläche zwischen den Kurven der arteriellen Compliance innerhalb eines transmuralen Druckbereichs von 0-100 mmHg berechnet und wobei der Berechnungsschritt den Unterschied zwischen der P-A-Grundlinienkurve und der hyperämischen P-A-Kurve als eine Fläche zwischen den P-A-Kurven innerhalb eines transmuralen Druckbereichs von 0-100 mmHg berechnet.

5. Verfahren nach Anspruch 4, wobei der Berechnungsschritt den Unterschied zwischen der Grundlinienkurve der arteriellen Compliance und der hyperämischen Kurve der arteriellen Compliance als eine Fläche zwischen den Kurven der arteriellen Compliance innerhalb eines transmuralen Druckbereichs von 20-80 mmHg berechnet und wobei der Berechnungsschritt den Unterschied zwischen der P-A-Grundlinienkurve und der hyperämischen P-A-Kurve als eine Fläche zwischen den P-A-Kurven innerhalb eines transmuralen Druckbereichs von 20-80 mmHg berechnet.

6. Verfahren nach Anspruch 1, wobei die Erzeugung der Grundlinienkurve der arteriellen Compliance und/oder einer Druck-Fläche (P-A)-Grundlinienkurve das Anwenden eines Bandpassfilters von 0,5-5,0 Hertz auf die Manschettendruckdaten, die während der Entleerungsphase der ersten segmentären Plethysmographie mit Manschette gesammelt werden, umfasst und wobei die Erzeugung der hyperämischen Kurve der arteriellen Compliance und/oder hyperämischen Druck-Fläche (P-A)-Kurve das Anwenden eines Bandpassfilters von 0,5-5,0 Hertz auf die Manschettendruckdaten, die während der Entleerungsphase der zweiten segmentären Plethysmographie mit Manschette gesammelt werden, umfasst.

**7.** Verfahren nach Anspruch 6, das weiter den Schritt des Anwendens eines zusätzlichen DC-Tiefpassfilters (0-0,5 Hz) auf Daten umfasst, die durch den Druckmessumformer während der Entleerungsphase der ersten und zweiten segmentären Plethysmographie mit Manschette erzeugt werden, um Durchflussdaten zu erzeugen.

**8.** Vorrichtung, die Folgendes umfasst:

eine aufblasbare Manschette, die einen Einlass und einen Auslass hat;
eine Pumpe (304), die mit dem Einlass der Manschette zum Aufblasen der Manschette verbunden ist;
einen Durchflussmesser, der mit dem Auslass der Manschette verbunden ist;
einen Druckmessumformer (306) zum Messen des Drucks innerhalb der Manschette; und
einen Computer (310), der für Folgendes konfiguriert ist;

Erzeugen einer Compliance-Kurve mit Manschette durch direktes Messen von Volumenänderung in der Manschette mit dem Durchflussmesser und von Druckänderung innerhalb der Manschette mit dem Druckmessumformer während eines Entleerungsvorgangs der Manschette;
Berechnen einer Kurve der arteriellen Compliance, die eine erste Kurve der arteriellen Compliance und eine zweite Kurve der arteriellen Compliance einschließt, und einer Druck-Fläche (P-A)-Kurve, die eine erste P-A-Kurve und eine zweite P-A-Kurve einschließt, nur während des Entleerungsvorgangs der Manschette; und
Berechnen eines Unterschieds zwischen Flächen unter der ersten Kurve der arteriellen Compliance und der zweiten Kurve der arteriellen Compliance und eines Unterschieds zwischen Flächen unter der ersten P-A-Kurve und der zweiten P-A-Kurve.

**9.** Vorrichtung nach Anspruch 8, wobei die Pumpe eine niederfrequente Pumpe ist, die eine Arbeitsfrequenz von 10 Hertz oder niedriger hat.

**10.** Vorrichtung nach Anspruch 8, wobei die Pumpe eine niederfrequente Pumpe ist, die eine Arbeitsfrequenz von 2 Hertz oder niedriger hat.

**Revendications**

**1.** Procédé de mesure de l'hyperémie réactive chez un patient, comprenant :

la réalisation d'une première pléthysmographie segmentaire par ballonnet ayant une phase de gonflage et une phase de dégonflage ;
la génération d'une courbe de compliance artérielle de référence et/ou d'une courbe pression-aire (P-A) de référence sur une partie du corps du patient, la pression du ballonnet étant augmentée jusqu'à une première pression maximale du ballonnet pendant la phase de gonflage et immédiatement réduite à partir de la première pression maximale du ballonnet pendant la phase de dégonflage ;
la réalisation d'une seconde pléthysmographie segmentaire par ballonnet ayant une phase de gonflage, une phase de maintien et une phase de dégonflage ;
la génération d'une courbe de compliance artérielle d'hyperémie et/ou d'une courbe pression-aire (P-A) d'hyperémie, la pression du ballonnet étant augmentée jusqu'à un second niveau maximal pendant la phase de gonflage, maintenue à la seconde pression maximale du ballonnet pendant une période prédéterminée pendant la phase de maintien, puis réduite à partir de la seconde pression maximale du ballonnet pendant la phase de dégonflage ;
le calcul de la différence entre la courbe de compliance artérielle de référence et la courbe de compliance artérielle d'hyperémie sous forme d'aire entre les courbes de compliance artérielle, et/ou de la différence entre la courbe P-A de référence et la courbe P-A d'hyperémie sous forme d'aire entre les courbes P-A ; et
la mesure d'un niveau d'hyperémie réactive d'après l'aire entre les courbes de compliance artérielle et/ou l'aire entre les courbes P-A,
une première courbe de compliance du ballonnet étant générée pendant la première pléthysmographie segmentaire par ballonnet d'après uniquement les données collectées pendant la phase de dégonflage de la première pléthysmographie segmentaire par ballonnet et une seconde courbe de compliance du ballonnet est générée pendant la seconde pléthysmographie segmentaire par ballonnet d'après uniquement les données collectées pendant la phase de dégonflage de la seconde pléthysmographie segmentaire par ballonnet et lesdites première et deuxième courbes de compliance du ballonnet étant générées à l'aide d'un débitmètre (302) qui mesure directement la variation de volume dans le ballonnet pendant la phase de dégonflage.

2. Procédé selon la revendication 1, la première pression maximale du ballonnet et la seconde pression maximale du ballonnet étant comprises dans la gamme de 150 à 180 mmHg.

3. Procédé selon la revendication 1 ou 2, la période prédéterminée étant de 2 à 10 minutes.

4. Procédé selon la revendication 1, l'étape de calcul calculant la différence entre la courbe de compliance artérielle de référence et la courbe de compliance artérielle d'hyperémie sous forme d'aire entre les courbes de compliance artérielle dans une gamme de pression transmurale de 0 à 100 mmHg, et l'étape de calcul calculant la différence entre la courbe P-A de référence et la courbe P-A d'hyperémie sous forme d'aire entre les courbes P-A dans une gamme de pression transmurale de 0 à 100 mmHg.

5. Procédé selon la revendication 4, l'étape de calcul calculant la différence entre la courbe de compliance artérielle de référence et la courbe de compliance artérielle d'hyperémie sous forme d'aire entre les courbes de compliance artérielle dans une gamme de pression transmurale de 20 à 80 mmHg, et l'étape de calcul calculant la différence entre la courbe P-A de référence et la courbe P-A d'hyperémie sous forme d'aire entre les courbes P-A dans une gamme de pression transmurale de 20 à 80 mmHg.

6. Procédé selon la revendication 1, la génération de ladite courbe de compliance artérielle de référence et/ou d'une courbe pression-aire (P-A) de référence comprenant l'application d'un filtre passe-bande de 0,5 à 5,0 Hertz aux données de pression du ballonnet collectées pendant la phase de dégonflage de ladite première pléthysmographie segmentaire par ballonnet et la génération de ladite courbe de compliance artérielle d'hyperémie et/ou d'une courbe pression-aire (P-A) d'hyperémie comprenant l'application d'un filtre passe-bande de 0,5 à 5,0 Hertz aux données de pression du ballonnet collectées pendant la phase de dégonflage de ladite seconde pléthysmographie segmentaire par ballonnet.

7. Procédé selon la revendication 6, comprenant en outre l'étape d'application d'un filtre passe-bas à CC supplémentaire (0 à 0,5 Hz) aux données générées par le capteur de pression pendant la phase de dégonflage desdites première et seconde pléthysmographies segmentaires par ballonnet pour générer des données de débit.

8. Appareil comprenant :

   un ballonnet gonflable ayant une entrée et une sortie ;
   une pompe (304) raccordée à ladite entrée dudit ballonnet pour gonfler le ballonnet ;
   un débitmètre raccordé à ladite sortie dudit ballonnet ;
   un capteur de pression (306) pour mesurer la pression à l'intérieur du ballonnet ; et
   un ordinateur (310) configuré pour :

   générer une courbe de compliance du ballonnet en mesurant directement la variation de volume dans ledit ballonnet avec ledit débitmètre et la variation de pression à l'intérieur du ballonnet avec ledit capteur de pression pendant un processus de dégonflage du ballonnet ;
   calculer une courbe de compliance artérielle, qui inclut une première courbe de compliance artérielle et une seconde courbe de compliance artérielle, et une courbe de pression-aire (P-A), qui inclut une première courbe P-A et une seconde courbe P-A, uniquement pendant le processus de dégonflage du ballonnet ; et
   calculer une différence entre les aires sous la première courbe de compliance artérielle et la seconde courbe de compliance artérielle et une différence entre les aires sous la première courbe P-A et la seconde courbe P-A.

9. Appareil selon la revendication 8, ladite pompe étant une pompe basse fréquence ayant une fréquence de fonctionnement de 10 Hertz ou moins.

10. Appareil selon la revendication 8, ladite pompe étant une pompe basse fréquence ayant une fréquence de fonctionnement de 2 Hertz ou moins.

FIG. 1

START

Baseline Data
Acquisition- Cuff
Inflation (Acknowledge)

*202*

Baseline Development of cuff
compliance mathematical formula-
Non-linear regression (SigmaPlot)

*204*

*200*

Baseline Data Acquisition- Cuff
Deflation (Acknowledge)

*206*

Baseline Development of Arterial
Compliance/P-A Curves/Flow Waveforms
(Use of Acknowledge and SigmaPlot)

*208*

*210*

Hyperemia Data
Acquisition- Cuff
Inflation
(Acknowledge)

Hyperemia Development of cuff
compliance mathematical formula- Non-
linear regression (SigmaPlot)

*212*

*214*

Hyperemia Data Acquisition- Cuff Deflation
(Acknowledge)

*216*

Hyperemia Development of Arterial Compliance/P-A
Curves/Flow Waveforms (Use of Acknowledge and
SigmaPlot)

*218*

Calculation of Metrics That Can Be Used in Patient
Monitoring (Acknowledge and SigmaPlot)

**FIG. 2**

END

Segment 1, 2:31:28 PM

FIG. 3

FIG. 4

FIG. 5

Patient 1-3 min Hyper
f=y0+(a/x)+(b/x^2)

FIG. 6

FIG. 7

Area between any normal and hyperemia curve will be tabulated. (Shaded area represents delta between baseline and reactive hyperemia.)

FIG. 8

EP 3 116 383 B1

To Release Valve

312

Flow Meter

306
Pressure
Transducer

Amplifier 308

To A/D Conversion, Signal
Processing, Data
Acquisition, Etc.

Blood Pressure
Cuff

Pressure Sensor 310

314

302

320

Pump

316

Pressure
Transducer

Temperature
Transducer

Computer

302

Pressure
Sensor

Temperature
Sensor

322

318

**FIG. 9**

Flow Meter

Rotary Pump

Single Board Computer

**FIG. 10**

SEGMENT 1, 2:31:28 PM

**FIG. 11**

**FIG. 12**

**FIG. 13**

**FIG. 14**

**FIG. 15**

**EP 3 116 383 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20473614
- US 2010305459 A1
- US 6309359 B1
- US 6309359 B